**Europäisches Patentamt**

**European Patent Office**

**Office européen des brevets**

(19)

(11) Veröffentlichungsnummer : **0 559 696 B1**

## (12) EUROPÄISCHE PATENTSCHRIFT

(45) Veröffentlichungstag der Patentschrift :
**20.07.94 Patentblatt 94/29**

(51) Int. Cl.⁵ : **A61K 7/50,** C11D 3/382, C09K 3/14

(21) Anmeldenummer : **91920456.0**

(22) Anmeldetag : **26.11.91**

(86) Internationale Anmeldenummer :
**PCT/EP91/02224**

(87) Internationale Veröffentlichungsnummer :
**WO 92/09265 11.06.92 Gazette 92/13**

(54) **ABRASIVUM IN KOSMETISCHEN PRODUKTEN UND VERFAHREN ZUR HERSTELLUNG UND VERWENDUNGEN DESSELBEN.**

(30) Priorität : **29.11.90 DE 4038076**

(43) Veröffentlichungstag der Anmeldung :
**15.09.93 Patentblatt 93/37**

(45) Bekanntmachung des Hinweises auf die
Patenterteilung :
**20.07.94 Patentblatt 94/29**

(84) Benannte Vertragsstaaten :
**AT BE CH DE DK ES FR GB GR IT LI LU NL SE**

(56) Entgegenhaltungen :
**WO-A-89/03669
CH-A- 564 349
US-A- 4 279 890
US-A- 4 407 789**

(73) Patentinhaber : **Chemische Fabrik
Stockhausen GmbH
Bäkerpfad 25
D-47805 Krefeld (DE)**

(72) Erfinder : **BOUILLON, Günter
Tulpenstr. 31
D-4152 Kempen (DE)**
Erfinder : **DANIEL, Günter
Krüllsdyk 132
D-4150 Krefeld (DE)**
Erfinder : **DENZER, Horst
Düsselstr. 24
D-4000 Düsseldorf (DE)**
Erfinder : **PEPPMÖLLER, Reinmar
Kemmerhofstr. 189
D-4150 Krefeld (DE)**
Erfinder : **FRANZEN, Martin
Langemarckstr. 2
D-4100 Duisburg 14 (DE)**

(74) Vertreter : **Klöpsch, Gerald, Dr.-Ing.
Patentanwalt
An Gross St. Martin 6
D-50667 Köln (DE)**

EP 0 559 696 B1

## Beschreibung

Die vorliegende Erfindung betrifft mit einem Bleichmittel, insbesondere Wasserstoffperoxid behandeltes Material in feiner Verteilung aus natürlichen Schalen und/oder Kernen, das als Abrasivum in kosmetischen Produkten enthalten ist, ein Verfahren zu seiner Herstellung sowie Verwendungen des Abrasivums.

Ein wesentlicher Bestandteil kosmetischer Reinigungs- und Behandlungsmittel ist das Reibemittel, das die Aufgabe hat, die Reinigungswirkung von waschaktiven bzw. tensidartigen Komponenten mechanisch zu unterstützen.

Nach dem Stand der Technik lassen sich zahlreiche anorganische und organische Materialien in Reinigungspräparaten als mechanische Reinigungs- und Behandlungsmittel einsetzen, beispielsweise in Handreinigern oder in sogenannten Peeling-Cremes und Spezialreinigungs-Gelen zur Entfernung der obersten, abgestorbenen Hautzellen oder von Hautverunreinigungen, beispielsweise im Gesicht oder an anderen Körperteilen.

In der EP-B-01 04 679 wird ein abrasives Material offenbart, das aus Agglomeraten von fein verteiltem Schleifmaterial besteht, wobei dieses mittels eines organischen Binders agglomeriert ist. Als Schleifmaterial wird Calzit verwendet. Zahlreiche andere abrasive Materialien sind bekannt, wie Kreide, Marmor, Dolomit, Feldspat oder Quarz. Aufgrund der starken Schleifwirkung sind diese Materialien jedoch für zahlreiche Anwendungen nicht geeignet. Im Falle des Calzit, der in sehr feiner Verteilung mit einem hydrophoben organischen Binder agglomeriert ist, läßt sich gemäß dieser Patentschrift das Zerkratzen empfindlicher Flächen vermeiden.

In Beispiel 4 wird der Zusatz eines chlorbildenden Bleichmittels erwähnt, wobei das Chlor die Reinigung des Reibemittels unterstützt und im Falle von zu reinigenden Kunststoff-Flächen keimtötend wirkt.

Die US-A-2561043 betrifft eine spezielle Vorrichtung, in der unter anderem aus Nußschalen Nußschalenmehle hergestellt werden können, ohne daß es beim Vermahlen zu Explosionen oder Feuern kommen kann.

Aus Nußschalen kann so ein < 325 mesh Nußschalenmehl (Maschenweite 45 μm) bei einer Betriebstemperatur der Vorrichtung von bis zu
275 °F (entsprechend 135 °C) erzeugt werden, indem das Mahlwerk mit 0,1 bis 0,5 Gallonen (entsprechend 0,4 bis 1,9 Liter) Wasser pro Minute gekühlt wird. Dieses Nußschalenmehl weist eine Restfeuchtigkeit von 2 bis 4 % auf.

Die US-A-4279890 betrifft Walnußschalenmehl enthaltende Puderzusammensetzungen, die Körperflüssigkeit gut absorbieren, und insbesondere aber die Gesichtspuderzusammensetzungen. Das Walnußschalenmehl, dessen Herstellung nicht näher erläutert ist, wird in Form eines Pulvers mit einer Teilchengröße von weniger als 40 μm, vorzugsweise von weniger als 30 μm eingesetzt, was nach Angaben dieses Standes der Technik eine zwingende Größe ist, um seine Eigenschaften als Füllstoff und ölabsorbierendes Agens zu sehen. Der Gehalt des Walnußschalenmehls in den kosmetischen Zusammensetzungen liegt zwischen 30 und 99 %, der Rest sind andere öl- und/oder feuchtigkeitsabsorbierende Füllstoffe, Bindemittel und weitere Hilfs- und Zusatzstoffe.

Die US-A 4,407,789 (E: Eigen et al.) betrifft eine stark absorbierende Körperpuderzusammensetzung, die im wesentlichen aus feingemahlenen Reisschalen besteht, die durch ein 200 Mesh-Sieb fallen, als Basispulver, in Mengen von bis zu 98 Gew.-%der Zusammensetzung und 1 bis 15 Gew.-% der Gesamtmischung eines geeigneten Bindemittels, das ausgewählt ist aus Zink-, Magnesium- und Aluminiumstearat. Die Aufgabe dieses US-Patents ist es daher, eine Körperpuderzusammensetzung zur Verfügung zu stellen, die eine hohe Absorptionsfähigkeit aufweist, sowie eine derartige Zusammensetzung bereitzustellen, die der Farbe der Körperhaut weitgehend angenähert ist. Auf das sehr hohe Absorptionsvermögen von getrockneten Reisschalen wird insbesondere in Spalte 2, Zeilen 41 bis 44 hingewiesen. Außer dem Hinweis auf den die Spalten 2 und 3 verbindenden Absatz, das die Reisschalen möglichst fein gemahlen werden sollen, ergeben sich keine weiteren Hinweise einer speziellen Behandlung dieses Reisschalenmehls, d.h. durch die Lehre des US-Patents wird nicht nahegelegt, die Schalen vor ihrer Verwendung zu bleichen.

In der japanischen Patentanmeldung J 611 59 495 wird eine Reinigungszusammensetzung offenbart, die Polysaccharid und Ethylen-Maleinsäureanhydrid-Copolymer enthält und als abrasives Mittel $SiO_2$, $Al_2O_3$, $MgO$, $SiC$, $B_4C$, $Fe_2O_3$, $TiO_2$ und andere sowie Zusatze wie Parfum, Farbstoffe, pilztötende Agenzien, Rostinhibitoren, Bleichmittel und pH-Kontrollmittel enthält.

Ähnliche abrasive Mittel werden in der japanischen Patentanmeldung J 59 036 200 beschrieben.

Um das Risiko zu starker Schleifwirkung anorganischer Materialien auszuschließen, ist Holzmehl als Schleifmittel in kosmetische Produkte eingeführt worden (Haut und Beruf, Strategien zu berufsbedingten Hauterkrankungen, H. Tronnier, Grosse-Verlag, Berlin 1989, Seiten 84/85).

Es ist jedoch seit langem bekannt, daß Holzstaub durch als Allergene wirkende Inhaltsstoffe nachteilige Wirkungen auf Haut und Atemwege haben kann. Holzinhaltsstoffe, die gesundheitliche Risiken darstellen, sind zum Beispiel Harze, Terpene, Phenole, Gerbstoffe und Chinone.

In anderen Produkten wird ein harz- und rindenfreies Weichholzmehl eingesetzt, das geringere Anteile an möglichen allergenen Begleitsubstanzen enthalten soll (Humane Produktion, 7/88, Seiten 8 und 9, Messebericht zur 9. Fachausstellung: Arbeitssicherheit in Hannover).

Eine Hautreinigungspaste, die Weichholzmehl und weitgehend unbehandeltes Olivenkerngranulat als Abrasiva enthält, wird in einer Werbung der Firma Chemische Fabrik Wilden GmbH, Postfach 10 11 80, 6078 Neu-Isenburg 1, beschrieben.

Holzmehl ist jedoch mit einem hohen Keimgehalt behaftet und als Folge seiner weichen, faserigen Struktur treten Quellprozesse im Endprodukt auf, die zu einem unerwünschten Viskositätsanstieg und damit zu gebrauchstechnischen Nachteilen führen. Ferner müssen ca. 2 Gew.-% an aufhellenden Pigmenten, wie zum Beispiel von Titandioxid zugesetzt werden, um helle, sauber wirkende und kosmetisch akzeptable Produkte zu erhalten.

Die genannten Nachteile des Holzmehls entfallen, wenn man Reibemittel auf Basis von Polyethylen- oder Polyurethan-Pulver einsetzt. Kunststoffpulver haben jedoch den Nachteil, daß sie biologisch nicht abbaubar sind und teilweise aufgrund schwierig durchzuführender Mahlprozesse zu einer unregelmäßigen Kornverteilung führen, so daß, auch bedingt durch das geringe Schüttgewicht dieser Materialien, formulierungstechnische Schwierigkeiten bei der Herstellung kosmetischer Produkte auftreten.

Als Materialien, die aufgrund ihrer Härte und Korngröße für die oberflächliche Hautreinigung geeignet sind, wurden natürliche Abrasivstoffe, wie gewaschene und gemahlene Schalen von Walnüssen sowie gemahlene Aprikosenkerne oder Olivenkerne eingeführt. Obgleich diese Materialien mit keimtötenden Mitteln behandelt werden, erreicht die maximale Keimzahl Werte von 200/g (siehe zum Beispiel Prospekt der Firma Cosmetochem AG, Riedstraße 7, CH-6330 Cham, Schweiz).

Diese Materialien haben eine schonende, sehr gut reinigende Wirkung ohne die Haut zu zerkratzen. Sie führen jedoch, wie auch Polyurethan-Pulver zu kosmetischen Produkten, die ein dunkles, schmutziges Aussehen haben. Man muß daher, wie bei Holzmehl aufhellende Pigmente, wie zum Beispiel Titandioxid mit ca. 2 Gew.-% zusetzen.

Ein Bleichverfahren für die Schalen von Walnüssen wird in Research and Industry, Volume 29, March 1984, Pages 10 - 16, beschrieben.

Durch eine Behandlung mit Natrium-Hydrogensulfit und anderen Mitteln soll das Aussehen der Schalen verbessert werden, um den Export der Walnüsse aus Indien nach Europa zu erleichtern.

Aus dem geschilderten Stand der Technik wird deutlich, daß noch immer der dringende Bedarf an einem Abrasivum besteht, das bei Einsatz in kosmetischen Reinigungsmitteln bei geringstmöglicher Konzentration oder unter völligem Verzicht an aufhellenden Substanzen zu optisch hellen und kosmetisch akzeptablen, sauberen Produkten führt. Es soll ein Naturprodukt sein und einen Keimgehalt unter 100/g besitzen, möglichst frei von allergenen Substanzen sowie biologisch abbaubar und von definierter, gleichbleibender Kornverteilung sein.

Der vorliegenden Erfindung liegt die Aufgabe zugrunde, einen abrasiven Stoff (Abrasivum) für kosmetische Präparate bereitzustellen.

Diese Aufgabe wird durch einen speziell erhaltenen und zusammengesetzten abrasiven Stoff gelöst. Die vorliegende Erfindung betrifft somit einen abrasiven Stoff für kosmetische Präparate, die ggfs. Tenside, Seifen oder andere Emulgatoren, organische Lösungsmittel oder Öle sowie ggfs. Verdickungsmittel, Gerüststoffe, Rückfettungsmittel, Parfümstoffe, Konservierungsmittel, Farbstoffe und Antioxidantien enthalten, der dadurch gekennzeichnet ist, daß er gebleichtes natürliches Schalen- und/oder Kernmehl enthält.

Weitere Aufgabe der Erfindung ist es, ein Herstellungsverfahren für diesen abrasiven Stoff bereitzustellen, das dadurch gekennzeichnet ist, daß natürliche Schalen und/oder Kerne zu einem Mehl definierter Korngröße gemahlen werden, das Mehl in wasserhaltiger Suspension mit Bleichmittel behandelt und anschließend abfiltriert wird und schließlich mit Wasser gewaschen und dann getrocknet wird.

Eine weitere Aufgabe der Erfindung ist dann, diesen abrasiven Stoff in Hautreinigungsmitteln zu verwenden. Schließlich ist es Aufgabe der Erfindung, kosmetische Zubereitungen, insbesondere Handwasch- und Handreinigungsmittel, bereitzustellen, die den vorgenannten abrasiven Stoff enthalten.

Bevorzugtes Bleichmittel ist wäßrige Wasserstoffperoxidlösung. Erfindungsgemäß ist die Herstellung des abrasiven Stoffes dadurch gekennzeichnet, daß natürliche Schalen und/oder Kerne zu einem Mehl definierter Kornverteilung gemahlen werden, das Mehl in wasserhaltiger Suspension mit Bleichmittel behandelt wird und anschließend abfiltriert, gewaschen und getrocknet wird.

Das Abrasivum wird erfindungsgemäß beispielsweise in lösungsmittelfreien oder lösungsmittelhaltigen Handwaschpasten, in wasserfreien Hautreinigungsmitteln und in Peeling-Cremes verwendet.

Das natürliche Schalen- oder Kernmehl kann beispielsweise Walnußschalenmehl, Mandelschalenmehl, Haselnußschalenmehl, Olivenkernmehl, Aprikosenkernmehl, Kirschkernmehl oder sonstiges natüriches Schalen- oder Kernmehl, beispielsweise von Palmkernen und Kokosnüssen, Pistazien- und Pinienschalen und anderem Kernobst sowie ein beliebiges Gemisch der genannten Materialien sein.

Um ein Produkt mit der gewünschten Kornverteilung zu erhalten, wird das Schalen- oder Kernmaterial in an sich bekannter Weise zu einem Mehl, ggfs. unter Einbeziehung einer Klassifizierung durch Siebe gemahlen, das eine Korngröße von 50 - 2000 μm, bevorzugt von 70 - 1000 μm und besonders bevorzugt von 80 - 400 μm besitzt.

Zum Mahlen können die bekannten Zerkleinerungsapparate bzw. Mühlen eingesetzt werden, wie zum Beispiel: Feinprallmühlen mit Pendel- oder Plattenschlagerwerk, Passagenwalzwerke, Hammerschlag- oder Stiftmühlen ggfs. mit Klassierungs-Aggregaten, z. B. Condux-mühlen.Das Mehl wird nunmehr in einem Rührbehälter bei einer Tempaertur von 20 °C bis 100 °C, vorzugsweise von 50 °C bis 100 °C, während 300 bis 30 Minuten, vorzugsweise 60 bis 90 Minuten, beispielhaft mit einer wäßrigen Lösung von Wasserstoffperoxid als Bleichmittel behandelt. Die Konzentration des Wasserstoffperoxid betragt 1,0 Gew.-% bis 10,0 Gew.-%, vorzugsweise 1,0 Gew.-% bis 3,0 Gew.-%, bezogen auf die gesamte Ansatzmenge. Vorzugsweise wird das Bleichmittel, z. B. das Wasserstoffperoxid in Kombination mit einem Stabilisierungsmittel bei der Bleichbehandlung eingesetzt. Im Falle einer Bleichbehandlung mit Wasserstoffperoxid kommen als Stabilisierungsmittel Natronwasserglas, Magnesiumchlorid, Carbonsäuren und Phosphorsäuren sowie Chelatbildner und Polyacrylsäure und/oder deren Salze in Frage.

Reste an $H_2O_2$ können ggfs. durch Reduktionsmittel, wie z. B. Natriumhydrogensulfit oder Ascorbinsäure bzw. durch Temperaturerhöhung der Suspension auf 100 °C zerstört werden.

Anschließend wird das Mehl abfiltriert und mit Wasser gewaschen, um Reste an wasserlöslichen Verunreinigungen zu entfernen. Das so erhaltene Produkt wird bei 100 °C bis 170 °C, vorzugsweise bei 120 °C bis 150 °C, oder bei Unterdruck im Temperaturbereich von 60 °C bis 140 °C bis zu einem Restfeuchtegehalt von 10 Gew.-%, vorzugsweise 0,2 bis 8,0 Gew.-%, getrocknet.

Das getrocknete Produkt kann nunmehr in unterschiedlichen kosmetischen Präparaten eingesetzt werden. Eine besonders geeignete Verwendung ist jedoch die Verwendung in Hautreinigungsmitteln.

Überraschend lassen sich mit einem solchen gebleichten Naturmehl unter volligem Verzicht bzw. mit nur 0,5 Gew.-% Titandioxid helle, saubere und kosmetisch akzeptable Pasten erhalten. Während vor dem Bleichen die Naturmehle Keimgehalte von $10^3$ bis $10^4$/g mitunter sogar mehr enthalten,erreicht man durch die Bleichbehandlung und Trocknungsprozeß solche von deutlich unter $10^2$/g. Dadurch lassen sich die in das Endprodukt einzusetzenden Konservierungsmittelmengen minimieren, was auch das durch Konservierungsmittel bedingte Allergisierungspotential verringert. Durch den Bleich- und Waschprozeß wird der Gehalt an unerwünschten Begleitstoffen im Mehl herabgesetzt. Die gebleichten Mehle sind biologisch vollständig abbaubar und liegen in definiert, gleichbleibender Kornverteilung vor. Verbunden mit einer höheren Festigkeit gegenüber dem Holzmehl läßt sich durch die einheitliche Kornverteilung eine den Handwaschpasten gebrauchsadäquate Viskositat erzielen, ohne daß Quellprozesse auftreten.

Die Prüfung von gebleichtem Walnußschalenmehl auf sensibilisierende Wirkung ergab keinen Hinweis auf eine Aktivierung des Immunsystems. Somit ist nicht zu erwarten, daß nach dermalem Kontakt bei menschlicher Exposition ein allergenes Potential besteht.

Mit Hilfe der Beispiele wird das Verfahren zur Herstellung und Anwendung des erfindungsgemäßen abrasiven Stoffes näher erläutert.

Beispiel 1

Bleichprozeß von natürlichem Schalen- oder Kernmehl mit Wasserstoffperoxid.

Der Bleichprozeß wird in einem 1000-Liter-Edelstahlapparat mit MIG-Rührwerk sowie Beheizungs- und Kühleinrichtung durchgeführt. Die Ansatzgröße betragt 518,3 kg.

In dem Reaktionsgefaß werden

| | |
|---|---|
| 300,0 kg | Wasser |
| 150,0 kg | Walnußschalenmehl, mittlere Korngröße 100 - 200 μm |
| 26,3 kg | Natronlauge 45%ig |
| 3,0 kg | Natronwasserglas und |
| 3,0 kg | Magnesiumchlorid |
| | vorgelegt. |

Die dunkelbraune Suspension wird unter stetigem Rühren auf 60 °C erwärmt. 26,3 kg Wasserstoffperoxid 35%ig werden über einen Zeitraum von 30 Minuten kontinuierlich zugegeben. Die Suspension wechselt nach der Hälfte der Zugabe die Farbe von dunkelbraun nach beige. Es wird zur Vollständigkeit der Reaktion 90 Minuten nachgerührt und der Rest-Wasserstoffperoxid-Gehalt titrimetrisch bestimmt.

Zur Reduktion des überschüssigen Wasserstoffperoxids kann die äquimolare Menge Ascorbinsaure oder Natriumsulfit zugesetzt werden, oder die Zersetzung wird durch Erwärmen auf 90 - 100°C erreicht.

Die Suspension wird über eine Kammerfilterpresse filtriert und der Filterkuchen mit heißem Wasser gewaschen. Der Wassergehalt beträgt danach ca. 50 %.

Das feuchte Naturmehl wird danach in einem Flugstromtrockner bis zu einem Restwassergehalt von 5,1 % getrocknet. Zur Trocknung kann auch ein Tellertrockner verwendet werden.

Die Ausbeute beträgt ca. 95 %, bezogen auf das eingesetzte Mehl.

In der getrockneten Form ist das gebleichte Mehl über mehrere Wochen und Monate lagerfähig, ohne mikrobiell zu verderben. Es kann so direkt in Handwaschpasten eingesetzt werden.

Beispiel 2

Bei der Optimierung des technischen Prozesses konnte die Menge des eingesetzten Magnesiumchlorids um 50 % und die Menge des zugegebenen Wasserstoffperoxids um 21,6 % reduziert werden. Bei einer Ansatzgröße von insgesamt 518,3 kg werden somit nur 1,5 kg Magnesiumchlorid und nur 20,6 kg Wasserstoffperoxid 35%ig benötigt, um das gleiche Bleichergebnis zu erhalten.

Eine weitere Verbesserung des Bleichverfahrens wurde dadurch erreicht, daß als Stabilisator Phosphorsäure statt Natronwasserglas und Magnesiumchlorid verwendet wird und der Prozeß bei Raumtemperatur abläuft. Das verbleibende Wasserstoffperoxid wird mit dem Waschwasser ausgewaschen bzw. beim Trocknungsprozeß zerstört. Die Vorteile liegen in der Einsparung an Prozeßzeit, Energiekosten und Rohstoffkosten. Der zur Reaktion benötigte Anteil an Wasserstoffperoxid konnte noch einmal um 25 %, bezogen auf die Einsatzkonzentration, reduziert werden.

In den Beispielen 3 bis 6 werden eine lösungsmittelfreie und eine lösungsmittelhaltige erfindungsgemäße Formulierung einer Handwaschpaste, eine Formulierung einer lösungsmittelfreien Handwaschpaste ohne $TiO_2$ bzw. mit geringem $TiO_2$- und Konservierungsmittelanteil, eines wasserfreien Hautreinigungsmittels und einer Peeling-Creme mit gebleichtem Schalen- und/oder Kernmehl angegeben.

Beispiel 3

Formulierung einer lösungsmittelfreien Handwaschpaste.

| Rohstoff | Einsatzmenge/Gew.-% |
|---|---|
| Tensid-Kombination bestehend aus Natrium-Laurylsulfat und Natrium-Alkylbenzolsulfonat, 50 %ig | 18,00 |
| Rizinusölsulfonat, 68 %ig | 10,00 |
| Wasser | 51,30 |
| Zitronensaure | 0,30 |
| Carboxymethylcellulose | 1,00 |
| Olein | 2,00 |
| Titandioxid | 2,00 |
| gebleichtes Schalen- oder Kernmehl z. B. Walnußschalenmehl < 350 µm | 15,00 |
| Hexamethylentetramin | 0,20 |
| Parfüm | 0,20 |
| | 100,00 |

Beispiel 4

Formulierung einer lösungsmittelfreien Handwaschpaste mit geringem Titandioxid- und Konservierungsmittelanteil.

| Rohstoff | Einsatzmenge/Gew.-% |
|---|---|
| Tensid-Kombination bestehend aus Natriumlaurylsulfat und Natriumalkylbenzolsulfonat, 50%ig | 18,00 |
| Rizinusölsulfonat, 68%ig | 10,00 |
| Wasser | 52,97 |
| Zitronensäure | 0,30 |
| Carboxymethylzellulose | 1,00 |
| Olein | 2,00 |
| Titandioxid | 0,50 |
| gebleichtes Schalen- oder Kern- mehl z. B. Walnußschalenmehl < 250 µm | 15,00 |
| 2-Brom-2-nitropropan-1,3-diol | 0,03 |
| Parfüm | 0,20 |
| | 100,00 |

Beispiel 5

Formulierung einer lösungsmittelfreien Handwaschpaste ohne Titandioxidanteil.

| Rohstoff | Einsatzmenge/Gew.-% |
|---|---|
| Tensid-Kombination bestehend aus Natriumlaurylsulfat und Natriumalkylbenzolsulfonat, 50%ig | 18,00 |
| Rizinusölsulfonat, 68%ig | 10,00 |
| Wasser | 53,47 |
| Zitronensäure | 0,30 |
| Carboxymethylzellulose | 1,00 |
| Olein | 2,00 |
| gebleichtes Schalen- oder Kern- mehl z. B. Olivenkernmehl < 400 µm | 15,00 |
| 2-Brom-2-nitropropan-1,3-diol | 0,03 |
| Parfüm | 0,20 |
| | 100,00 |

Beispiel 6

Formulierung einer lösungsmittelhaltigen Handwaschpaste.

| Rohstoff | Einsatzmenge/Gew.-% |
|---|---|
| Natrium-Monoethanol- | |
| aminfettalkohol-Sulfat, 40%ig | 37,00 |
| Rizinusölsulfonat, 68%ig | 14,00 |
| Wasser | 11,97 |
| Zitronensäure | 0,30 |
| Carboxymethylzellulose | 2,00 |
| n-Paraffin | 19,00 |
| Siliziumdioxid | 0,50 |
| gebleichtes Schalen- oder | |
| Kernmehl z. B. Walnußschalenmehl < 250 µm | 15,00 |
| 2-Brom-2-nitropropan-1,3-diol | 0,03 |
| Parfüm | 0,20 |
| | 100,00 |

Für die Formulierung der Handwaschpasten können auch andere Tenside, wie zum Beispiel Natrium-Fettalkohol-Ethersulfat, Cocamidopropylbetain oder Alkylpolyglykoside oder Abmischungen davon verwendet werden.

Die Herstellung der Produkte erfolgt nach den üblichen, bekannten Verfahren, die allgemein für die Formulierung von Tensid-Systemen bekannt sind (G. Ziolkowski, Kosmetik-Jahrbuch 1986, 1987, 1989, Verlag für Chemische Industrie, H. Ziolkowski KG Augsburg; W. Umbach, Kosmetik, 1988, Georg-Thieme-Verlag Stuttgart, insbesondere Kapitel 5 und 13).

Beispiel 7

Formulierung eines wasserfreien Hautreinigungsmittels.

| Rohstoff | Einsatzmenge/Gew.-% |
|---|---|
| Fettalkohol $C_{12}$-$C_{15}$ EU (waschaktive Substanzen) | 15,0 |
| Dimethyladipat (Lösemittel) | 9,3 |
| Dimethylglutarat (Lösemittel) | 35,3 |
| Dimethylsuccinat (Lösemittel) | 9,4 |
| iso-Octylstearat (Rückfettungsmittel) | 5,0 |
| pyrogene Kieselsäure (Verdickungsmittel) | 2,3 |
| Ethylenglykoldistearat (Verdickungsmittel) | 7,0 |
| Celluloseacetobutyrat (Verdickungsmittel) | 3,8 |
| Parfüm | 0,4 |
| Walnußschalenmehl < 250 µm, gebleicht | 12,5 |
| | 100,0 |

Beispiel 8

Formulierung einer Peeling-Creme.

7

A. MIGLYOL<sup>R</sup> 812 Neutraiol (gemiscnt säuriges
   Triglycerid der fraкt. C8-10 Kokosfettsäuren;
   Herst.: Dynamit Nobel AG)                                66,45%
   IMWITOR<sup>R</sup> 780 K (Herst.: Dynamit Nobel AG)         5,0 %
   Teginacid<sup>R</sup> (Mischung von Glycerinmonodistearaten mit Anteilen an Polyglykolfettalkohoietnern; Herst.: TH. Goldschmidt AG)            3,4 %
   Texapon<sup>R</sup> L 100 (Natriumlaurylsulfat, Herst.:
   Henkel KG aA)                                             1,45 %
   Paraffinöl                                                2,5  %
   Phenonip                                                  1,0  %
   (Phenoxiethanol mit Paqraвenen, Nipa Laboratories, GB)

B. Kartoffeistarкe                                          5,0  %
   Olivenkernmehl < 300 μm, geвleicht                        6,0  %

C. Aerosil<sup>R</sup> 200 (Pyroiytiscn nergesteiltes hochdisperses Siliciumaioxid Herst.: Degussa AG)                4,0  %
   Syloid<sup>R</sup> 244 (hochdiserse Kieseisäure;
   Herst.: Grace and Co.)                                    6,0  %
   Parfümöl                                                  0,2  %
                                                           ──────
                                                           100,00%

Beispiel 9

Konservierungsmittelversuch anhand einer Formulierung einer lösungsmittelfreien Handwaschpaste.

| Rohstoff | Einsatzmenge/Gew.-% |
|---|---|
| Tensid-Kombination besteнend aus Na-Laurylethersulfat, Cocamiаopropyl-betain und Rizinusoisulfonat (40%ig) | 57,9 |
| gebleichtes bzw. ungebleichte Mischung aus Walnußschaien und Olivenkernmenl, 50 : 50, < 300 μm | 15,0 |
| Verdicker-Kombination aus Carboxymethylceiiuiose und Bentonit | 5,0 |
| Titandioxid | 0,5 |
| Zitronensäure | 0,2 |
| Parfüm | 0,2 |
| Konservierungsmittei it. Tabelle | |
| Wasser | ad 100,0 |

8

## Tabelle

| Konzentration des Konservierungsmittels (Bronopol 2-Brom-2-nitropropan-1,3-diol) | 0,00 % | 0,03 % | 0,08 % |
|---|---|---|---|
| Keime/g bei Einsatz der ungebleichten Mischung (nach 2 Tagen) | 13.000 (Wachstum) | 2,400 (Wachstum) | 200 (Wachstum) |
| Keime/g bei Einsatz der gebleichten Mischung (nach 2 Tagen) | < 100 | < 100 | < 100 |

Bei Einsatz der ungebleichten Schalen-Kernmehlmischung in einer lösemittelfreien Handwaschpaste ohne Konservierungsmittel nach Rezeptur Beispiel 9 wurden im Produkt nach 2 Tagen Keimzahlen von $1,3 \times 10^4$/g gefunden, während das Produkt bei Verwendung des gebleichten Mehles keine erhöhten Keimzahlen aufwies.

## Patentansprüche

1. Abrasiver Stoff für kosmetische Präparate, insbesondere Hautreinigungsmittel, die ggfs. Tenside, Seifen oder andere Emulgatoren, organische Lösungsmittel oder Öle sowie ggfs. Verdickungsmittel, Gerüststoffe, Rückfettungsmittel, Parfümstoffe, Konservierungsmittel, Farbstoffe und Antioxidantien enthalten, dadurch gekennzeichnet, daß er gebleichtes natürliches Schalen- und/oder Kernmehl enthält.

2. Abrasiver Stoff nach Anspruch 1, dadurch gekennzeichnet, daß das Schalen- und/oder Kernmehl eine definierte Korngröße besitzt.

3. Abrasiver Stoff nach den Ansprüchen 1 und 2, dadurch gekennzeichnet, daß er aus mit wäßrigem Wasserstoffperoxid gebleichtem, feinteiligem Schalen- und/oder Kernmehl besteht.

4. Abrasiver Stoff nach den Ansprüchen 1 bis 3, dadurch gekennzeichnet, daß die Korngröße 50 - 2000 µm, vorzugsweise 70 - 1000 µm und besonders bevorzugt 80 - 400 µm beträgt.

5. Abrasiver Stoff nach den Ansprüchen 1 bis 4, dadurch gekennzeichnet, daß als natürliche Schalen- oder Kernmehle Walnußschalen-, Mandelschalen- und Haselnußschalenmehl, Oliven-, Aprikosen- und Kirschkernmehl oder beliebige Gemische dieser Schalen- und Kernmehle verwendet werden.

6. Abrasiver Stoff nach den Ansprüchen 1 bis 5, dadurch gekennzeichnet, daß er in wasserfreie bzw. wasserhaltige Hautreinigungsmittel eingesetzt wird.

7. Abrasiver Stoff nach den Ansprüchen 1 bis 6, dadurch gekennzeichnet, daß er einen Gehalt an gebleichten, feinteiligen, natürlichen Schalen- und/oder Kernmehlen von 3 - 95 Gew.-%, vorzugsweise von 5 - 70 Gew.-%, besonders bevorzugt von 7 - 50 Gew.-% enthält, bezogen auf das Gesamtgewicht des Hautreinigungsmittels.

8. Abrasiver Stoff nach den Ansprüchen 1 bis 7, dadurch gekennzeichnet, daß Schalen- und/oder Kernmehl in einer Korngröße von 50 - 2000 µm, vorzugsweise von 70 - 1000 µm, besonders bevorzugt von 80 - 400 µm eingesetzt wird.

9. Verfahren zur Herstellung des abrasiven Stoffes nach den Ansprüchen 1 bis 8, dadurch gekennzeichnet, daß natürliche Schalen und/oder Kerne zu einem Mehl definierter Korngröße gemahlen werden, das Mehl in wasserhaltiger Suspension mit Bleichmittel behandelt und anschließend abfiltriert, mit Wasser gewaschen und getrocknet wird.

10. Verfahren nach Anspruch 9, dadurch gekennzeichnet, daß das feinteilige Schalen- und/oder Kernmehl mit wäßrigem Wasserstoffperoxid als Bleichmittel behandelt wird.

11. Verfahren nach Anspruch 9 oder 10, dadurch gekennzeichnet, daß das Bleichmittel weiterhin ein Stabilisierungsmittel enthält.

12. Verfahren zur Herstellung eines abrasiven Stoffes nach den Ansprüchen 9, 10 oder 11, dadurch gekennzeichnet, daß Schalen- und/oder Kernmehl eingesetzt wird, das auf eine Korngröße von 50 - 2000 μm, vorzugsweise von 70 - 1000 μm und besonders bevorzugt von 80 - 400 μm gemahlen wird.

13. Verfahren zur Herstellung eines abrasiven Stoffes nach den Ansprüchen 9 bis 12, dadurch gekennzeichnet, daß die Behandlung mit Wasserstoffperoxid bei einer Temperatur von 20 °C bis 100 °C, vorzugsweise von 50 °C bis 100 °C, besonders bevorzugt bei 20 °C bis 50 °C erfolgt.

14. Verfahren zur Herstellung eines abrasiven Stoffes nach den Ansprüchen 9 bis 13, dadurch gekennzeichnet, daß die Behandlung mit Wasserstoffperoxid während einer Verweilzeit von 300 bis 30 Minuten, vorzugsweise von 60 bis 30 Minuten erfolgt.

15. Verfahren zur Herstellung eines abrasiven Stoffes nach den Ansprüchen 9 bis 14, dadurch gekennzeichnet, daß das Wasserstoffperoxid in der wäßrigen Suspension eine Konzentration von 1,0 bis 10,0 Gew.-%, vorzugsweise von 1,0 bis 3,0 Gew.-% besitzt.

16. Verfahren zur Herstellung eines abrasiven Stoffes nach den Ansprüchen 9 bis 15, dadurch gekennzeichnet, daß das gebleichte und gewaschene Material bei 100 °C bis 170 °C, vorzugsweise bei 120 °C bis 150 °C, bevorzugt bei Unterdruck im Temperaturbereich von 60 °C bis 140 °C, getrocknet wird.

17. Verfahren zur Herstellung eines abrasiven Stoffes nach den Ansprüchen 9 bis 16, dadurch gekennzeichnet, daß die Trocknung bis zu einer Restfeute von 10,0 Gew.-%, vorzugsweise von 0 bis 8,0 Gew.-% erfolgt.

18. Verwendung des abrasiven Stoffes nach den Ansprüchen 1 bis 8 in wasserfreien bzw. wasserhaltigen Hautreinigungsmitteln enthaltend Tenside, Seifen oder andere Emulgatoren, organische Lösungsmittel oder Öle sowie gegebenenfalls Verdickungsmittel, Gerüststoffe, Farbstoffe und Antioxidantien, gekennzeichnet durch einen Gehalt an gebleichtem, feinteiligem, natürlichem Schalen- und/oder Kernmehl als Reibemittel zur Unterstützung der Hautreinigung.

19. Verwendung des abrasiven Stoffes nach Anspruch 18 in Hautreinigungsmitteln, dadurch gekennzeichnet, daß als natürliches Schalen- und/oder Kernmehl Walnuß-, Haselnuß- oder Mandelschalenmehl bzw. Oliven-, Aprikosen- oder Kirschkernmehl oder beliebige Gemische derselben verwendet werden.

20. Verwendung des abrasiven Stoffes nach den Ansprüchen 18 und 19 in Hautreinigungsmitteln, gekennzeichnet durch einen Gehalt an gebleichten, feinteiligen, natürlichen Schalen- und/oder Kernmehlen von 3 - 95 Gew.-%, vorzugsweise von 5 - 70 Gew.-%, besonders bevorzugt von 7 - 50 Gew.-%, bezogen auf das Gesamtgewicht.

21. Verwendung des abrasiven Stoffes nach den Ansprüchen 18 bis 20 in Handwaschpasten.

22. Kosmetische Zubereitungen, insbesondere Handwaschmittel und Handreinigungsmittel, enthaltend einen abrasiven Stoff gemäß den Ansprüchen 1 bis 8, oder der hergestellt ist nach den Ansprüchen 9 bis 17.

## Claims

1. An abrasive for cosmetic preparations, in particular skin cleaning agents, which optionally comprise surfactants, soaps or other emulsifiers, organic solvents or oils and, optionally, thickeners, builders, re-

EP 0 559 696 B1

greasing agents, perfumes, preservatives, dyes and antioxidants, characterized in that it comprises bleached natural shell and/or kernel flour.

2. The abrasive according to claim 1 characterized in that said shell and/or kernel flour has a defined particle size.

3. The abrasive according to claims 1 and 2 characterized in that it consists of finely divided shell and/or kernel flour bleached with aqueous hydrogen peroxide.

4. The abrasive according to claims 1 to 3 characterized in that the particle size amounts to 50 - 2000 μm, preferably 70 - 1000 μm, and most preferably to 80 to 400 μm.

5. The abrasive according to claims 1 to 4 characterized in that the flour of walnut shells, almond shells and hazelnut shells, that of olive kernels, apricot and cherry stones, or any mixtures of said shell and stone flours is used as said natural shell or kernel flours.

6. The abrasive according to claims 1 to 5 characterized in that it is used in anhydrous or hydrous skin cleaning agents.

7. The abrasive according to claims 1 to 6 characterized in that it comprises a content of bleached, finely divided, natural shell and/or kernel flours amounting to 3 - 95%-wt., preferably 5 - 70%-wt., and most preferably 7 - 50%-wt., relative to the total weight of the skin cleansing agent.

8. The abrasive according to claims 1 to 7 characterized in that shell and/or kernel flour is used having a particle size of 50 - 2000 μm, preferably 70 - 1000 μm, and most preferably of 80 - 400 μm.

9. A process for the production of the abrasive as defined in claims 1 to 8 characterized in that natural shells and/or kernels are ground into a flour having a defined particle size, that the flour is treated with a bleaching agent in aqueous suspension, is subsequently filtered off, washed with water and dried.

10. The process according to claim 9 characterized in that said finely divided shell and/or kernel flour is treated with aqueous hydrogen peroxide as bleaching agent.

11. The process according to claim 9 or 10 characterized in that said bleaching agent additionally comprises a stabilizer.

12. The process for the manufacture of an abrasive according to claims 9, 10, or 11 characterized in that shell and/or kernel flour is used which is ground to a particle size of 50 - 2000 μm, preferably 70 - 1000 μm, and most preferably 80 - 400 μm.

13. The process for the production of an abrasive according to claims 9 to 12 characterized in that said treatment with hydrogen peroxide is effected at a temperature of 20°C to 100°C, preferably 50°C to 100°C, and most preferably at 20°C to 50°C.

14. The process for the production of an abrasive according to claims 9 to 13 characterized in that said treatment with hydrogen peroxide is effected during a residence time of 300 to 30 minutes, preferably of 60 to 30 minutes.

15. The process for the production of an abrasive according to claims 9 to 14 characterized in that said hydrogen peroxide in said aqueous suspension has a concentration of 1.0 to 10.0% by weight, preferably of 1.0 to 3.0% by weight.

16. The process for the production of an abrasive according to claims 9 to 15 characterized in that the bleached and washed material is dried at 100°C to 170°C, preferably at 120°C to 150°C, more preferably under vacuum within a temperature range of 60°C to 140°C.

17. The process for the production of an abrasive according to claims 9 to 16 characterized in that drying is effected up to a residual moisture content of 10.0%-wt., preferably of 0 to 8.0%-wt.

18. The use of the abrasive as defined in claims 1 to 8 in anhydrous or hydrous skin cleansing agents comprising surfactants, soaps or other emulsifiers, organic solvents or oils, as well as, optionally, thickeners,

11

builders, dyestuffs, and antioxidants, characterized by a content of bleached, disperse, natural shell and/or kernel flour as abrasive to support skin cleaning.

19. The use of the abrasive according to claim 18 in skin cleansing agents characterized in that the flour of walnut shells, hazelnut shells or almond shells, or that of olive kernels, apricot or cherry stones, or any mixtures thereof, is used as said natural shell and/or kernel flour.

20. The use of the abrasive according to claims 18 and 19 in skin cleaning agents characterized by a content of bleached, finely divided, natural shell and/or kernel flours amounting to 3 - 95%-wt., preferably 5 - 70%-wt., and most preferably 7 - 50%-wt., relative to the total weight.

21. The use of the abrasive according to claims 18 to 20 in hand cleaning pastes.

22. Cosmetic preparations, in particular hand detergents and hand cleaning agents, comprising an abrasive according to claims 1 to 8 or one manufactured according to claims 9 to 17.


## Revendications

1. Produit abrasif pour des préparations cosmétiques, plus particulièrement des compositions de nettoyage de la peau, qui contiennent éventuellement des agents tensioactifs, des savons, ou d'autres émulsifs, des solvants organiques, ou des huiles, comme éventuellement aussi des agents épaississants, des adjuvants de détergence ou de nettoyage, des agents renourrissants, des parfums, des conservateurs, des colorants et des antioxydants, caractérisé en ce qu'il contient de la farine d'écales et/ou de noyaux naturelle, blanchie.

2. Produit abrasif suivant la revendication 1, caractérisé en ce que la farine d'écales et/ou de noyaux possède un calibre des grains défini.

3. Produit abrasif suivant les revendications 1 et 2, caractérisé en ce qu'il se compose de farine d'écales et/ou de noyaux, blanchie à l'aide de peroxyde d'hydrogène aqueux.

4. Produit abrasif suivant les revendications 1 à 3, caractérisé en ce que le calibre des grains varie de 50 à 2000 $\mu$m, de préférence 70 à 1000 $\mu$m et, plus avantageusement encore, de 80 à 400 $\mu$m.

5. Produit abrasif suivant les revendications 1 à 4, caractérisé en ce que l'on utilise, à titre de farines d'écales ou de noyaux naturelles, de la farine d'écales de noix, d'écales d'amandes et d'écales de noisettes, de noyaux d'olives, de noyaux d'abricots et de noyaux de cerises, ou n'importe quels mélanges de ces farines d'écales et de noyaux.

6. Produit abrasif suivant les revendications 1 à 5, caractérisé en ce qu'on l'utilise dans des agents de nettoyage de la peau dépourvus d'eau ou contenant de l'eau.

7. Produit abrasif suivant les revendications 1 à 6, caractérisé en ce qu'il possède une teneur en farines d'écales et/ou de noyaux naturelles, finement divisées, blanchies, de 3 à 95% en poids, de préférence 5 à 70% en poids, mieux encore de 7 à 50% en poids, par rapport au poids total de l'agent de nettoyage de la peau.

8. Produit abrasif suivant les revendications 1 à 7, caractérisé en ce qu'on utilise la farine d'écales et/ou de noyaux d'un calibre des grains de 50-2000 $\mu$m, de préférence 70-1000 $\mu$m, plus avantageusement encore, 80-400 $\mu$m.

9. Procédé de préparation du produit abrasif suivant les revendications 1 à 8, caractérisé en ce que l'on broie ou mout les écales et/ou les noyaux naturels en une farine à calibre des grains défini, on traite la farine en suspension contenant de l'eau par un agent de blanchiment et on la sépare ensuite par filtration, on la lave à l'eau et on la sèche.

10. Procédé suivant la revendication 9, caractérisé en ce que l'on traite la farine d'écales et/ou de noix, finement divisée, par du peroxyde d'hydrogène aqueux à titre d'agent de blanchiment.

11. Procédé suivant la revendication 9 ou 10, caractérisé en ce que l'agent de blanchiment contient en outre un agent de stabilisation.

12. Procédé de préparation d'un produit abrasif suivant la revendication 9, 10 ou 11, caractérisé en ce que l'on utilise une farine d'écales et/ou de noix qui a été broyée ou moulue jusqu'à un calibre des grains de 50-2000 μm, de préférence 70-1000 μm et, de manière particulièrement avantageuse, de 80-400 μm.

13. Procédé de préparation d'un produit abrasif suivant les revendications 9 à 12, caractérisé en ce que le traitement par le peroxyde d'hydrogène s'effectue à une température de 20°C à 100°C, de préférence de 50°C à 100°C, mieux encore, de 20°C à 50°C.

14. Procédé de préparation d'un produit abrasif suivant les revendications 9 à 13, caractérisé en ce que le traitement par le peroxyde d'hydrogène s'effectue en l'espace d'une durée de séjour de 300 à 30 minutes, de préférence de 60 à 30 minutes.

15. Procédé de préparation d'un produit abrasif suivant les revendications 9 à 14, caractérisé en ce que le peroxyde d'hydrogène possède, dans la suspension aqueuse, une concentration de 1,0 à 10,0% en poids, de préférence de 1,0 à 3,0% en poids.

16. Procédé de préparation d'un produit abrasif suivant les revendications 9 à 15, caractérisé en ce que la matière blanchie et lavée est séchée à une température de 100°C à 170°C, de préférence de 120°C à 150°C et de manière plus avantageuse encore, sous pression réduite dans la plage des températures de 60°C à 140°C.

17. Procédé de préparation d'un produit abrasif suivant l'une quelconque des revendications 9 à 16, caractérisé en ce que le séchage s'effectue jusqu'à une teneur en humidité résiduelle de 10,0% en poids, de préférence 0 à 8,0% en poids.

18. Utilisation du produit abrasif suivant les revendications 1 à 8 dans des compositions de nettoyage de la peau dépourvues d'eau ou contenant de l'eau, qui contiennent des agents tensioactifs, des savons ou d'autres émulsifs, des solvants organiques, ou des huiles, comme éventuellement aussi des agents épaississants, des adjuvants de détergence ou de nettoyage, des colorants et des antioxydants, caractérisés par une teneur en farine d'écales et/ou de noyaux naturelle, finement divisée, blanchie, servant d'agent abrasif ou de friction destiné à soutenir ou favoriser le nettoyage de la peau.

19. Utilisation du produit abrasif suivant la revendication 18 dans des compositions de nettoyage de la peau, caractérisée à ce qu'à titre de farine d'écales et/ou de noyaux naturelle, on utilise de la farine d'écales de noix, de noisettes ou d'amandes, ou de la farine de noyaux d'olives, d'abricots ou de cerises, ou n'importe quels mélanges de ces farines.

20. Utilisation du produit abrasif suivant les revendications 18 et 19 dans des compositions de nettoyage de la peau, caractérisées par une teneur en farines d'écales et/ou de noyaux naturelles, finement divisées, blanchies, de 3 à 95% en poids, de préférence 5 à 70% en poids, mieux encore de 7 à 50% en poids, par rapport au poids total.

21. Utilisation du produit abrasif suivant les revendications 18 à 20 dans des pâtes de lavage des mains.

22. Préparations cosmétiques, plus particulièrement compositions de lavage des mains et compositions de nettoyage des mains, contenant un produit abrasif suivant les revendications 1 à 8, ou qui a été préparé suivant les revendications 9 à 17.